# EUROPEAN PATENT SPECIFICATION

(11) **EP 3 027 185 B1**
(45) Date of publication and mention of the grant of the patent: **22.01.2020**
(21) Application number: 14761908.4
(22) Date of filing: 29.07.2014
(51) Int. Cl.: A61K 31/445, C07D 211/46, A01H 1/04, A01H 5/10

(54) **CHARACTERIZATION OF RICE**
CHARAKTERISIERUNG VON REIS
CARACTÉRISATION DU RIZ

(30) Priority: 29.07.2013 GB 201313440
(43) Date of publication of application: 08.06.2016
(73) Proprietor: Phytoquest Limited, Aberystwyth SY23 3EB (GB)
(72) Inventor: NASH, Robert James, Aberystwyth SY23 3EB (GB)
(74) Representative: EIP
(86) International application number: PCT/GB2014/052319
(87) International publication number: WO 2015/015186

(56) References cited:
- RODRIGUEZ-SANCHEZ S ET AL: "Improvement of a gas chromatographic method for the analysis of iminosugars and other bioactive carbohydrates", JOURNAL OF CHROMATOGRAPHY A, vol. 1289, May 2013 (2013-05), pages 145-148, XP002732073,
- SUSANA AMÃ CR ZQUETA ET AL: "Determination of-fagomine in buckwheat and mulberry by cation exchange HPLC/ESIâ Q-MS", ANALYTICAL AND BIOANALYTICAL CHEMISTRY, SPRINGER, BERLIN, DE, vol. 402, no. 5, 30 December 2011 (2011-12-30), pages 1953-1960, XP035009720, ISSN: 1618-2650, DOI: 10.1007/S00216-011-5639-2
- NAKAGAWA K ET AL: "Determination of iminosugars in mulberry leaves and silkworms using hydrophilic interaction chromatography-tandem mass spectrometry", ANALYTICAL BIOCHEMISTRY, ACADEMIC PRESS INC, NEW YORK, vol. 404, no. 2, 15 September 2010 (2010-09-15), pages 217-222, XP027132890, ISSN: 0003-2697 [retrieved on 2010-05-12]
- NASH ROBERT J ET AL: "Iminosugars as therapeutic agents: recent advances and promising trends", FUTURE MEDICINAL CHEMISTRY, FUTURE SCIENCE LTD, GB, vol. 3, no. 12, 1 September 2011 (2011-09-01), pages 1513-1521, XP009181092, ISSN: 1756-8919, DOI: 10.4155/FMC.11.117
- IVAN A ROSS: "11. Oryza sativa", MEDICINAL PLANTS OF THE WORLD. VOLUME 3: CHEMICAL CONSTITUENTS, TRADITIONAL AND MODERN MEDICAL USES,, vol. 3, 1 January 2005 (2005-01-01), pages 401-418, XP009181082,
- KATO ATSUSHI ET AL: "2,5-Dideoxy-2,5-imino-D-altritol as a new class of pharmacological chaperone for Fabry disease", BIOORGANIC & MEDICINAL CHEMISTRY, vol. 18, no. 11, June 2010 (2010-06), pages 3790-3794, XP002732074,
- IKEDA K ET AL: "Homonojirimycin analogues and their glucosides from Lobelia sessilifolia and Adenophora spp. (Campanulaceae)", CARBOHYDRATE RESEARCH, PERGAMON, GB, vol. 323, no. 1-4, 12 January 1999 (1999-01-12), pages 73-80, XP004186502, ISSN: 0008-6215, DOI: 10.1016/S0008-6215(99)00246-3
- RODRIGUEZ-SANCHEZ SONIA ET AL: "New Methodologies for the Extraction and Fractionation of Bioactive Carbohydrates from Mulberry (Morus alba) Leaves", JOURNAL OF AGRICULTURAL AND FOOD CHEMISTRY, vol. 61, no. 19, May 2013 (2013-05), pages 4539-4545, XP002732075,
- KATO A ET AL: "Fagomine isomers and glycosides from Xanthocercis zambesiaca", JOURNAL OF NATURAL PRODUCTS, AMERICAN CHEMICAL SOCIETY, US, vol. 60, no. 3, 1 March 1997 (1997-03-01), pages 312-314, XP002621630, ISSN: 0163-3864, DOI: 10.1021/NP960646Y
- MICHALIK A ET AL: "Steviamine, a new indolizidine alkaloid from Stevia rebaudiana", PHYTOCHEMISTRY LETTERS, ELSEVIER, AMSTERDAM, NL, vol. 3, no. 3, 20 September 2010 (2010-09-20), pages 136-138, XP027275211, ISSN: 1874-3900 [retrieved on 2010-04-24]
- KATO ET AL: "Iminosugars from Baphia nitida Lodd", PHYTOCHEMISTRY, PERGAMON PRESS, GB, vol. 69, no. 5, 14 January 2008 (2008-01-14), pages 1261-1265, XP022495608, ISSN: 0031-9422, DOI: 10.1016/J.PHYTOCHEM.2007.11.018
- NOJIMA H ET AL: "Antihyperglycemic effects of N-containing sugars from Xanthocercis zambesiaca, Morus bombycis, Aglaonema treubii, and Castanospermum australe in streptozotocin-diabetic mice", JOURNAL OF NATURAL PRODUCTS, AMERICAN CHEMICAL SOCIETY, US, vol. 61, no. 3, 1 March 1998 (1998-03-01), pages 397-400, XP002621629, ISSN: 0163-3864, DOI: 10.1021/NP970277L [retrieved on 1998-02-20]

## Description

### Field of the Invention

This invention relates to processes for producing rice-based compositions, methods for monitoring the quality of rice, and for the selection, breeding, identification and screening of rice strains and varieties which find utility in the treatment of energy utilization diseases.

### Background of the Invention

Rice is the seed of the monocot plants *Oryza sativa* (Asian rice) or *Oryza glaberrima* (African rice). There are four major categories of rice: indica (nonsticky, long-grained); japonica (sticky, short grained); aromatic (medium- to long-grained) known for its nut-like aroma and taste, and glutinous (also called sticky rice), which has opaque grains.

Varieties of aromatic rice include: basmati, jasmine and wild pecan rice. When cooked, the grains have a light and fluffy texture. Basmati rice is aromatic rice grown in India and Pakistan. It has a nut-like taste and has a buttery flavour. It is considered long-grain white rice and is lower in starch than other varieties. Cooked grains of Basmati rice are characteristically free flowing rather than sticky, as with most long-grain rice. In addition to normal (white) Basmati rice, brown basmati is available. Basmati rice has been viewed as a healthier option than white rice because of its lower glycaemic index (GI) value.

Varieties of japonica rice include Arborio rice and Baldo rice, cultivars grown in Italy.

Rice is also classified on the basis of colour, including white, brown, black, purple and red varieties. Brown rice is produced when the seeds of the rice plant are milled using a rice huller to remove the chaff (the outer husks of the grain). The milling may be continued, removing the bran (the rest of the husk and the germ), thereby creating white rice. White rice has a relatively long shelf-life, but lacks some important nutrients. Rice bran contains many bioactive components with disease fighting properties including the capacity to modulate gut microbiotica.

A group at Colorado State University is currently studying how rice bran augments mucosal immunity (Kumar, A. et al. 2012, BMC Microbiology, 12, 71). Rice bran has been found to control gastrointestinal cancers, hyperlipidaemia and diabetes in rats (Cheng, H.H. et al., 2010, International journal for vitamin and nutrition research Internationale Zeitschrift fur Vitamin- und Ernahrungsforschung, 80(1):45-53; Tomita, H. et al., 2008, Oncol Rep 2008, 19(1):11-15) as well as hypercholesterolemia in humans (Gerhardt, A. & Gallo, N. 1998, J Nutr., 128(5):865-869).

Rice has long been known to have a range of health benefits. The ancient literatures of rice-growing Asian countries such as Thailand, China, Malaysia, Indonesia and India have attributed medicinal properties to rice, in addition to it being the mainstay as food. According to the Ayurvedic treaties of Charaka (c.700BC) and Susruta (c.400BC), rice has medicinal value in the treatment of various ailments such as diarrhea, vomiting, fever, haemorrhage, chest pain, wounds and burns. Even to this day, certain varieties are still used in some parts of India to treat skin diseases, blood pressure, fever, paralysis and rheumatism. The famous Nivara rice of Kerala is widely used in Ayurvedic practice for body enrichment, to exclude toxic metabolites, strengthen, revitalize and energise the body, regulate blood pressure, prevent skin diseases and delay premature ageing (Ahuja, U., et al. 2008, Asian Agri-History 12, 93-108).

In China, the medicinal value of rice was known as far back as 2,800BC, when it was used by royal Chinese physicians for healing purposes. The Chinese believe that rice strengthens the spleen as well as stomach, increases appetite and cures indigestion. In Malaysia dried powdered rice is recommended for skin ailments, whilst in Cambodia, the hulls of mature plants are considered useful in treating dysentery.

Positive qualities associated with rice include the high digestibility of its starch, high biological value of amino acids, high content of essential fatty acids and selenium. Rice-based oral rehydration solutions (ORS) are preferred to glucose-based ORS in the treatment of acute diarrhoea (Gore S., et al. 1992, British Medical Journal 304, 287-291) and have been included in WHO recommendations. Rice is a low allergenic food and is recommended for people who have irritable bowel syndrome (Ahuja U. et al, 2008, Asian Agri-History 12, 93-108).

Some rice varieties are claimed to have a lower Glycemic index (GI) than others. The glycaemic index or GI is a measure of the effects of carbohydrates in food on blood sugar levels. It estimates how much each gram of available carbohydrate (total carbohydrate minus fiber) in a food raises a person's blood glucose level following consumption of the food, relative to consumption of glucose (Jenkins, D. et al., 1981, Am J Clin Nutr 34, 362-366). Glucose has a glycaemic index of 100, by definition, and other foods have a lower glycaemic index. Foods with carbohydrates that break down quickly during digestion and release glucose rapidly into the bloodstream tend to have a high GI; foods with carbohydrates that break down more slowly, releasing glucose more gradually into the bloodstream, tend to have a low GI. A lower glycaemic index suggests slower rates of digestion and absorption of the foods' carbohydrates and may also indicate greater extraction from the liver and periphery of the products of carbohydrate digestion. A lower glycaemic response usually equates to a lower insulin demand but not always, and may improve long-term blood glucose control (Jenkins, D. et al. 2008, JAMA. 300, 2742-2753) and blood lipids.

A systematic review of published reports (Barclay et al, 2009, American Journal of Clinical Nutrition, 87, 627-637) concluded that low GI diets are independently associated with a reduced risk of certain chronic diseases including diabetes and heart disease. In diabetic patients, evidence from medium-term studies suggests that replacing high-glycaemic-index carbohydrates with low-glycaemic-index forms will improve glycaemic control and, among persons treated with insulin, will reduce hypoglycaemic episodes (Willett, W. et al., 2002, American Journal of Clinical Nutrition, 76, 274S-280S).

Coloured rices have been extensively studied and their anthocyanin and flavonoid content associated with anti oxidant properties (Zhang, M. et al., 2010, J. Agric. Food Chem., 58, 7580-7587). Red and black rices were shown to reduce atherosclerotic plaque formation by 50% more than white rice in rabbits (Ling, W. et al. 200,: Journal of Nutrition, 131, 1421-1426). In a recent study (Das, S. and De, B., 2012, Nutrition & Food Science, 42, 428-433), the enzyme inhibitory properties of red rice have been compared with those of white varieties. Aqueous methanol extracts of a number of varieties of both types were tested against α-amylase and α-glucosidase enzymes. The white varieties showed weak α-glucosidase inhibition and no α-amylase inhibition whereas the red varieties showed good α-amylase and α-glucosidase inhibition. Although the anthocyanins in coloured rice are reported to have α-glucosidase inhibitory activities (Akkarachiyasit, S. et al., 2010, Int. J. Mol. Sci., 11, 3387-3396), the authors here suggest that some other component may also contribute to the inhibition observed. In another study, black rice extracts have been reported to be better α-glucosidase inhibitors than those from red rice (Yao, Y. et al., 2010, J. Agric. Food Chem., 58, 770-774).

### Energy utilization diseases

Energy utilization diseases encompass a wide range of diseases and include, for example, disorders of homeostasis, metabolic diseases, dysfunction of sugar metabolism and appetite disorders.

Examples of energy utilization diseases therefore include insulin resistance, various forms of diabetes, metabolic syndrome, obesity, wasting syndromes (for example, cancer associated cachexia), myopathies, gastrointestinal disease, growth retardation, hypercholesterolemia, atherosclerosis and age-associated metabolic dysfunction.

Energy utilization diseases also include conditions associated with metabolic syndrome, obesity and/or diabetes, including for example hyperglycaemia, glucose intolerance, hyperinsulinaemia, glucosuria, metabolic acidosis, cataracts, diabetic neuropathy, diabetic nephropathy, diabetic retinopathy, macular degeneration, glomerulosclerosis, diabetic cardiomyopathy, insulin resistance, impaired glucose metabolism, arthritis, hypertension, hyperlipidemia, osteoporosis, osteopenia, bone loss, brittle bone syndromes, acute coronary syndrome, infertility, short bowel syndrome, chronic fatigue, eating disorders and intestinal motility dysfunction.

### Insulin resistance, metabolic syndrome and diabetes

In healthy individuals, blood glucose levels are maintained within a narrow range by two pancreatic hormones: insulin (produced by pancreatic β-cells) and glucagon (produced by pancreatic α-cells). Pancreatic β-cells sense increases in blood glucose levels and respond by secreting insulin. Insulin promotes glucose uptake by tissues of the body, thereby restoring blood glucose concentration to the physiological range. Glucagon acts reciprocally, increasing blood glucose levels under fasting conditions, primarily by stimulating glucose production in the liver.

Insulin resistance is characterized by a reduced action of insulin in skeletal muscle, adipocytes and hepatocytes so that normal amounts of insulin become inadequate to produce a normal insulin response from the cells of these tissues. In adipocytes, insulin resistance results in hydrolysis of stored triglycerides, leading to elevated free fatty acids in the blood plasma. In muscle, insulin resistance reduces glucose uptake while in hepatocytes it reduces glucose storage. In both of the latter cases an elevation of blood glucose concentrations results.

High plasma levels of insulin and glucose due to insulin resistance often progresses to metabolic syndrome and type 2 diabetes.

Metabolic syndrome is a constellation of abnormalities and disorders that increase the risk of cardiovascular disease and diabetes. The incidence is very high in many developed countries: some studies indicate prevalence in the USA of up to 25% of the population. The disorder is also known as (metabolic) syndrome X, insulin resistance syndrome, Reaven's syndrome and CHAOS. Metabolic syndrome may be diagnosed by the presence of three or more of the following symptoms: central obesity (waist measurement of more than 40 inches for men and more than 35 inches for women); high levels of triglycerides (150 mg/dL or higher); low levels of HDL (below 40 mg/dL for men and below 50 mg/dL for women) and high blood pressure (130/85 mm Hg or higher). Associated diseases and signs are: fatty liver (often progressing to non-alcoholic fatty liver disease), polycystic ovarian syndrome, hemochromatosis (iron overload) and acanthosis nigricans (dark skin patches).

The first line treatment of metabolic syndrome is change of lifestyle (caloric restriction and physical activity). However, drug treatment is frequently required. Generally, the individual diseases that comprise the metabolic syndrome are treated separately (e.g. diuretics and ACE inhibitors for hypertension). Cholesterol drugs may be used to lower LDL cholesterol and triglyceride levels, if they are elevated, and to raise HDL levels if they are low. Use of drugs that decrease insulin resistance (e.g. metformin and thiazolidinediones is controversial). Cardiovascular exercise is therapeutic in less than 31% of cases and does not generally produce a decrease in fasting plasma glucose or insulin resistance.

Thus, new and/or alternative treatments for metabolic syndrome are required, particularly treatments which are effective against obesity and/or elevated triglyceride levels.

Type 2 diabetes is a chronic disease that is characterised by persistently elevated blood glucose levels (hyperglycaemia). Insulin resistance together with impaired insulin secretion from the pancreatic β-cells characterizes the disease. The progression of insulin resistance to type 2 diabetes is marked by the development of hyperglycaemia after eating when pancreatic β-cells become unable to produce adequate insulin to maintain normal blood sugar levels (euglycemia).

The most important drug currently used to treat type 2 diabetes is metformin (Glucophage, Diabex, Diaformin, Fortamet, Riomet, Glumetza, Cidophage and others). Metformin is of the biguanide class of oral antihyperglycaemic agents. Other biguanides include phenformin and buformin (now withdrawn). Metformin works primarily by reducing liver release of blood glucose from glycogen stores, but also has some effect in increasing the uptake of glucose. Other widely used drug classes include those of the sulfonylurea group (including glibenclamide and gliclazide). These drugs increase glucose stimulated insulin secretion by the pancreas. Newer drug classes include thiazolidinediones (e.g. rosiglitazone, pioglitazone, and troglitazone), which act by binding to PPARs (peroxisome proliferator-activated receptors), a group of receptor molecules inside the cell nucleus. Other classes include α-glucosidase inhibitors (acarbose), meglitinides (which stimulate insulin release and include nateglinide, repaglinide and their analogues), peptide analogues (e.g. incretin mimetics, which act as insulin secretagogues, glucagon-like peptide analogues (e.g. exenatide), dipeptidyl peptidase-4 (DPP-4) inhibitors (which increase incretin levels (e.g. sitagliptin) and amylin agonist analogues (which slow gastric emptying and suppress glucagon (e.g. pramlintide).

However, no existing therapies for the different forms of type 2 diabetes seem to improve function of key intrinsic factors in the β-cells and all existing therapies fail to arrest progression of the disease and, over time, also fail to normalise glucose levels and/or prevent subsequent complications. The existing therapies are also associated with undesirable side effects. For example, insulin secretagogues and insulin injections may cause hypoglycaemia and weight gain. Patients may also become unresponsive to insulin secretagogues over time. Metformin and α-glucosidase inhibitors often lead to gastrointestinal problems and PPAR agonists tend to cause increased weight gain and oedema. Exenatide is also reported to cause nausea and vomiting.

Glycosylation has an important role in regulating properties of proteins and is associated with many diseases. Itoh, N. et al., 2007 (Am J Physiol Endocrinol Metab 293: E1069-E1077) have reported the serum *N*-glycan profile in human subjects with type 2 diabetes and found an increased amount of a biantennary *N*-glycan that had an α1,6-fucose with a bisecting *N*-acetylglucosamine. Copeland, R.J. et al., 2008 (Am J Physiol Endocrinol Metab 295: E17-E28) have reviewed the importance of O-linked N-acetylglucosamine in diabetes and concluded that there is a strong positive correlation between GlcNAcylation and the development of insulin resistance. *O*-linked -β-*N*-acetylglucosamine (*O*-GlcNAc) is a dynamic posttranslational modification that, analogous to phosphorylation, cycles on and off serine and/or threonine hydroxyl groups. Cycling of *O*-GlcNAc is regulated by the concerted actions of *O*-GlcNAc transferase and *O*-GlcNAcase. GlcNAcylation is involved in the aetiology of glucose toxicity and chronic hyperglycemia-induced insulin resistance, a major hallmark of type 2 diabetes. Hexosaminidase activity has been shown to be elevated in the serum of diabetics (e.g. Agardh, C.D. et al., 1982, Acta Med Scand. 212:39-41).

Type 1 diabetes (or insulin dependent diabetes) is characterized by loss of the insulin-producing beta cells of the islets of Langerhans in the pancreas, leading to a deficiency of insulin. The main cause of this beta cell loss is a T-cell mediated autoimmune attack. There is no known preventative measure that can be taken against type 1 diabetes, which comprises up to 10% of diabetes mellitus cases in North America and Europe. Most affected people are otherwise healthy and of a healthy weight when onset occurs. Sensitivity and responsiveness to insulin are usually normal, especially in the early stages.

The principal treatment of type 1 diabetes, even from the earliest stages, is replacement of insulin combined with careful monitoring of blood glucose levels using blood-testing monitors. Without insulin, ketosis and diabetic ketoacidosis can develop and coma or death will result. Apart from the common subcutaneous injections, it is also possible to deliver insulin by a pump, which allows continuous infusion of insulin 24 hours a day at preset levels, and the ability to program doses (a bolus) of insulin as needed at meal times. An inhaled form of insulin, Exubera, has recently been approved by the FDA.

The treatment of type 1 diabetes must be continued indefinitely. While treatment does not impair normal activities, great awareness, appropriate care, and discipline in testing and medication must be observed.

Thus, new and/or alternative antidiabetic drug treatments, particularly those that are able to restore β-cell function, are required. In particular, there is a real and substantial unmet clinical need for an effective drug that is capable of treating both type 2 and type 1 diabetes and associated conditions with fewer side effects than existing drug therapies.

### Iminosugars

Iminosugars are a class of sugar mimics in which the endocyclic oxygen of sugars is substituted by a basic nitrogen atom. Many are naturally-occurring, constituting a class of alkaloids present as secondary metabolites in the tissues of certain plants (where they may play a role in defence).

Iminosugars have been shown to have diverse biological properties (often arising from their structural resemblance to the sugar moieties of natural substrates, which in many cases is reflected in inhibitory activity against various enzymes, including glucosidases). Indeed, iminosugars are now recognized as a profoundly important class of therapeutic agents (see Iminosugars From Synthesis to Therapeutic Applications: Compain, Philippe & Martin, Olivier R. (eds.) ISBN-13: 978-0-470-03391-3 - John Wiley & Sons). Indeed, humans have been using iminosugars (typically in the form of plant extracts) as poisons, narcotics, stimulants and medicines for thousands of years.

The therapeutic applications of polyhydroxylated alkaloids have been comprehensively reviewed in Watson, A.A. et al. 2001, Phytochemistry 56: 265-295: applications include cancer therapy, immune stimulation, the treatment of diabetes, the treatment of infections (especially viral infections), therapy of glycosphingolipid lysosomal storage diseases and the treatment of autoimmune disorders (such as arthritis and sclerosis).

Structurally, iminosugars exhibit great diversity. They may be classified structurally on the basis of the configuration of the N-heterocycle. Examples of some important alkaloids and their structures are set out in Kutchan,T.M. 1995, The Plant Cell 7:1059-1070, while Watson, A.A. et al., 2001 Phytochemistry 56: 265-295 have classified a comprehensive range of iminosugars *inter alia* as piperidine, pyrroline, pyrrolidine, pyrrolizidine, indolizidine and nortropanes alkaloids (see Figs. 1-7 of Watson, A.A. et al. (2001))

Watson, A.A. et al. (2001), *ibidem* also show that a functional classification of at least some alkaloids is possible on the basis of their glycosidase inhibitory profile: many polyhydroxylated alkaloids are potent and highly selective glycosidase inhibitors. These alkaloids can mimic the number, position and configuration of hydroxyl groups present in pyranosyl or furanosyl moieties and so bind to the active site of a cognate glycosidase, thereby inhibiting it. This area is reviewed in Legler, G., 1990, Adv. Carbohydr. Chem. Biochem. 48: 319-384 and in Asano, N. et al., 1995, J. Med. Chem. 38: 2349-2356.

### Summary of the Invention

The present invention is based, at least in part, on the surprising discovery of iminosugars in certain rice varieties. Thus, rice may be classified on the basis of its iminosugar profile: iminosugars (including certain bioactive iminosugars) occur in some, but not all, rice varieties.

The newly-discovered rice iminosugars include bioactive iminosugars which inhibit glucose absorption and have therapeutic activity in the treatment of energy utilization diseases, but may also reduce the nutritional value of the rice. They may also contribute to pest/pathogen resistance of the botanic source, and may have cosmetic applications.

Thus, this discovery has profound implications for the utilization of the global rice crop, and implies the need to classify rice on the basis of its iminosugar profile so that the strain or variety appropriate to intended use (e.g. famine relief, treatment of malnutrition, management of energy utilization diseases, crop pest resistance, cosmetic application etc.) can be selected.

Significantly, the newly-discovered rice iminosugars include the iminosugars fagomine (D-fagomine, FG, 1, 2-dideoxynojirimycin) and 3,4,5-trihydroxy-2-hydroxymethyl tetrahydropyridine (1-deoxynojirimycin, DNJ):

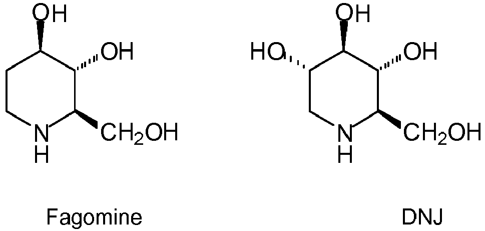

DNJ is known to inhibit glucose absorption in the small intestine by inhibiting disaccharidases and attenuating the expression of proteins involved in the transepithelial glucose transport system, and maintains stable blood glucose levels by directly regulating the expression of enzyme proteins involved in hepatic glycolysis and gluconeogenesis. It has been proposed as a therapeutic option for diabetic disease (see Li, Y. et al. 1-deoxynojirimycin inhibits glucose absorption and accelerates glucose metabolism in streptozotocin-induced diabetic mice. Sci. Rep. 3, 1377; DOI:10.1038/srep01377 (2013). Mulberry extract containing DNJ has been shown to control weight gain in rodents and to suppress lipid accumulation (Kong, W. H. et al., 2008, J. Agric. Food Chem. 56: 2613-2619).

Fagomine was originally identified in buckwheat (*Fagopyrum esculentum* Moench, Polygonaceae) and has since been found in other plant families, including the *Moraceae, Fabaceae* and *Solanaceae,* as a minor iminosugar. Buckwheat is present in the human diet all around the world and has a reputation for being a healthy food. Fagomine lowers postprandial blood glucose and modulates bacterial adhesion (Gómez, L. et al., 2012, D-Fagomine lowers postprandial blood glucose and modulates bacterial adhesion. British Journal of Nutrition, 107, pp 1739-1746. It has been proposed as a dietary ingredient or functional food component to reduce the health risks associated with an excessive intake of fast-digestible carbohydrates, or an excess of potentially pathogenic bacteria (ibid.). Fagomine has been shown in rodent pancreatic cell studies to potentiate glucose induced-insulin secretion (Watson, A. A., et al., 2001, Polyhydroxylated Alkaloids - Natural Occurrence and Therapeutic Applications. Phytochemistry 56: 265-295).

Without wishing to be bound by any theory, the inventors postulate that the newly-discovered rice iminosugars, including *inter alia* fagomine and DNJ, contribute to lowering blood sugar, lowering GI index and controlling weight gain. They may also control the levels of sugars in the skin, and inhibit melanin formation. Thus, the invention finds application in the selection, breeding, identification and screening of rice strains and varieties which find utility as pest/pathogen resistant crops, in the treatment of energy utilization diseases (as described above), the treatment of malnutrition, in famine relief operations and as cosmetics (including in particular compositions for topical application to the skin).

Again without wishing to be bound by any theory, the inventors also postulate that the presence of iminosugars in rice may lower its nutritional value, so that rice varieties intended for use in foodstuffs and food supplements for famine relief or for treating malnutrition should be screened for iminosugar content, and selected to avoid strains and varieties which contain iminosugars which reduce nutritional value.

Thus, according to the invention there is provided a method for selecting breeding lines and/or varieties of plants of the genus Oryza as a botanical source of foodstuffs of low or high GI index, the method comprising the steps of: (a) providing seeds or vegetative parts from plants of the genus Oryza; and (b) determining the presence or absence and/or measuring the amount of at least one polyhydroxylated alkaloid iminosugar in the seeds or vegetative parts of step (a).

In another aspect, there is provided the use of rice material from seeds of plants of the genus Oryza as a food or feed supplement to lower the glycaemic index (GI) value of said food or feed, wherein the rice material is selected from indica rice, japonica rice, aromatic rice, glutinous rice, brown rice, white rice, black rice, purple rice, red rice, rice bran, nonsticky rice, sticky rice, long-grained rice, short-grained rice or medium-grained rice and contains at least one endogenous polyhydroxylated alkaloid rice iminosugar, wherein said rice material is selected for a high content of said endogenous rice iminosugar.

Other aspects and preferred embodiments of the invention are defined and described in the claims set out below.

### Detailed Description of the Invention

### Definitions

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

The phrase "consisting essentially of" is used herein to require the specified integer(s) or steps as well as those which do not materially affect the character or function of the claimed invention.

As used herein, the term "consisting" is used to indicate the presence of the recited integer (e.g. a feature, element, characteristic, property, method/process step or limitation) or group of integers (e.g. features, element, characteristics, properties, method/process steps or limitations) alone.

The term *iminosugar* is a term of art which defines a saccharide analogue in which the ring oxygen is replaced by a nitrogen. The term *polyhydroxylated alkaloid* as used herein defines a class of oxygenated iminosugars. Typically these have at least 2, 3, 4, 5, 6 or 7 (preferably 3, 4 or 5) hydroxyl groups (or alkyl groups with one or more hydroxy substituent(s)) on the ring system nucleus. The term iminosugar is used herein to include iminosugar acids. The term *iminosugar acid* defines a sugar acid analogue in which the ring oxygen is replaced by a nitrogen.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, pathological variegated states). In this case, the term is used synonymously with the term "therapy". Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

The term "subject" (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals and pet animals. In preferred embodiments, the subject is a human.

As used herein, an *effective amount* of a compound or composition defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

The term "metabolic syndrome" is used herein to define conditions characterized by the presence of three or more of the following symptoms: central obesity (waist measurement of more than 40 inches for men and more than 35 inches for women); high levels of triglycerides (150 mg/dL or higher); low levels of HDL (below 40 mg/dL for men and below 50 mg/dL for women) and high blood pressure (130/85 mm Hg or higher).

The term therefore includes conditions defined in accordance with the definition of metabolic syndrome by the World Health Organization: (a) fasting plasma glucose above 6.1 mmol/L; (b) blood pressure above140/90 mm Hg; and (c) one or more of the following: (i) plasma triglycerides above 1.7mmol/L; (ii) HDL below 0.9 and 1.0 mmol/L (for men and women, respectively); (iii) a body mass index above 30 kg/m².

References herein to the treatment of metabolic syndrome are to be interpreted to include the treatment of any or all of the disorders associated with metabolic syndrome, including in particular obesity (e.g. central obesity) and elevated serum triglycerides.

References herein to the treatment of type 1 or type 2 diabetes are to be interpreted to include the treatment of type 1 and type 2 diabetes *per se* as well as pre-diabetes (incipient diabetes) and insulin resistance.

The term "pre-diabetes" or "incipient diabetes" defines conditions in which elevated levels of glucose or glycosylated haemoglobin are present in the absence of diabetes.

### Medical uses of the invention

The health benefits of controlling sugar in blood and tissues and reducing weight are numerous including controlling diabetes, metabolic syndrome, slowing deterioration of the cardiovascular system and slowing ageing of the skin. The iminosugars have many potential benefits to health including Th-1 immune response modulation, anti-inflammatory activity, anti-cancer and anti-viral activity and show chaperoning of proteins improving functionality (Nash, R.J., et al., 2011, Iminosugars as therapeutic agents: recent advances and promising trends. Future Med. Chem. 3 (12): 1513-1521). Iminosugars also have potential to control insect and other pests of plants and so their presence may also be related to degrees of pest and pathogen resistance of rice varieties (Nash, R.J., et al., 1996, Polyhydroxylated alkaloids that inhibit glycosidases. In: Alkaloids: Chemical and Biological Perspectives. Vol.11. S.W. Pelletier (ed). Elsevier Science Ltd. Oxford, pp.345-376).

The invention finds broad application in the treatment of malnutrition and in any energy utilization disease.

Thus, diseases which may be treated according to the invention include, for example, disorders of homeostasis, metabolic diseases, dysfunction of sugar metabolism and appetite disorders.

In preferred embodiments, the invention finds application in the treatment of insulin resistance, various forms of diabetes, metabolic syndrome, obesity, wasting syndromes (for example, cancer associated cachexia), myopathies, gastrointestinal disease, growth retardation, hypercholesterolemia, atherosclerosis and age-associated metabolic dysfunction.

The invention may also be used for the treatment of conditions associated with metabolic syndrome, obesity and/or diabetes, including for example hyperglycaemia, glucose intolerance, hyperinsulinaemia, glucosuria, metabolic acidosis, cataracts, diabetic neuropathy, diabetic nephropathy, diabetic retinopathy, macular degeneration, glomerulosclerosis, diabetic cardiomyopathy, insulin resistance, impaired glucose metabolism, arthritis, hypertension, hyperlipidemia, osteoporosis, osteopenia, bone loss, brittle bone syndromes, acute coronary syndrome, infertility, short bowel syndrome, chronic fatigue, eating disorders, intestinal motility dysfunction and sugar metabolism dysfunction.

The invention may also be used to suppress appetite.

Particularly preferred is the treatment of insulin resistance, metabolic syndrome, obesity and diabetes (particularly type 2 diabetes).

### Insulin resistance, metabolic syndrome and diabetes

The invention finds application in the treatment of insulin resistance. Insulin resistance is characterized by a reduced action of insulin in skeletal muscle, adipocytes and hepatocytes so that normal amounts of insulin become inadequate to produce a normal insulin response from the cells of these tissues. In adipocytes, insulin resistance results in hydrolysis of stored triglycerides, leading to elevated free fatty acids in the blood plasma. In muscle, insulin resistance reduces glucose uptake while in hepatocytes it reduces glucose storage. In both of the latter cases an elevation of blood glucose concentrations results. High plasma levels of insulin and glucose due to insulin resistance often progresses to metabolic syndrome and type 2 diabetes.

The invention finds application in the treatment of metabolic syndrome (as herein defined). The disorder is also known as (metabolic) syndrome X, insulin resistance syndrome, Reaven's syndrome and CHAOS.

The invention finds application in the treatment of diseases associated with metabolic syndrome, including for example: fatty liver (often progressing to non-alcoholic fatty liver disease), polycystic ovarian syndrome, hemochromatosis (iron overload) and acanthosis nigricans (dark skin patches).

The invention finds application in the treatment of Type 2 diabetes. Type 2 diabetes is a chronic disease that is characterised by persistently elevated blood glucose levels (hyperglycaemia). Insulin resistance together with impaired insulin secretion from the pancreatic β-cells characterizes the disease. The progression of insulin resistance to type 2 diabetes is marked by the development of hyperglycaemia after eating when pancreatic β-cells become unable to produce adequate insulin to maintain normal blood sugar levels (euglycemia).

### Type 1 diabetes

The invention finds application in the treatment of Type 1 diabetes (or insulin dependent diabetes). Type 1 diabetes is characterized by loss of the insulin-producing beta cells of the islets of Langerhans in the pancreas, leading to a deficiency of insulin. The main cause of this beta cell loss is a T-cell mediated autoimmune attack. There is no known preventative measure that can be taken against type 1 diabetes, which comprises up to 10% of diabetes mellitus cases in North America and Europe. Most affected people are otherwise healthy and of a healthy weight when onset occurs. Sensitivity and responsiveness to insulin are usually normal, especially in the early stages.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### Example 1: Iminosugars in a Black Rice

A black rice from Italy (Gallo Venere Riso Nero - sell by date 26/04/15 - 8 001 1420 002782) was extracted in boiling water as normal for cooking and also a sample ground and extracted in cold 50% aq. Ethanol. Both extracts were filtered and fractionated on the strongly acidic cation exchange resin IR120 in the H⁺ form. After washing with water the retained material was displaced with 2M ammonia solution and reduced in volume by Rotary evaporation. The IR120 retained fraction of the cold extracted material was then further fractionated into a bound and unbound sample on an anion exchange resin (CG400 OH⁻ form). All fractions were assayed against a panel of glycosidases and they showed inhibition of α- and β-glucosidases which could be due to fagomine and DNJ but additionally they strongly inhibited α- and β--galactosidase and two N-acetyl-β-D-glucosaminidases which would not be caused by fagomine or DNJ.

Analysis of the fractions by Gas chromatography (GC-MS) by the method described showed a complex mixture of iminosugars comprising 16% of the low molecular weight amino acid fraction (retained by IR120 resin). 8.5mg/100g of the rice appeared to be fagomine plus several other iminosugars, some probably novel (not matching reported structures by mass spectra). Fagomine gives a distinctive mass spectrum as the trimethylsilylderivative with major fragments observed at 144, 170, 260 and 348 amu. Other iminosugars in this rice were characterized by distinctive iminosugar fragments at 170, 262, 286 and 376 amu. An epimer of fagomine (possibly 3-epifagomine) was also observed with similar spectrum but shorter retention time (6.47 minutes compared to 7.2 minutes for fagomine).

### GC-MS

All samples were freeze dried before derivatisation. Trimethylsilyl (TMS) derivatives were prepared using a mixture of hexamethyldisilazane and trimethylchlorosilane in pyridine (Pierce 'Tri-Sil' silylation reagent, HMDS:TMCS:pyridine in a ratio of 2:1:10). Samples were heated at 60°C for 15 minutes and then left at room temperature for at least 60 min. Insoluble reaction products were sedimented by centrifugation, and the supernatant was transferred to fresh vials using a syringe.

Analysis was carried out by GC-MS using a Perkin Elmer Autosystem XL gas chromatograph with a high polarity fused-silica column (Varian 'Factor Four' VF-5ms column, 25 m x 0.25 mm i.d., 0.25 µm phase thickness). The carrier gas (helium) flow rate was 1 ml min-1. Trimethylsilyl- (TMS) derivatives were separated using a temperature programme that started at 160°C for 5 min, followed by a linear increase to 300°C at a rate of 10°C min-1. The temperature was held at 300°C for an additional 10 min; the total analysis time was 29 min. Electron impact mass spectrometry of the column eluant was carried out using a Perkin Elmer TurboMass Gold mass spectrometer, with a quadrupole ion filter system, which was run at 250°C constantly during analysis. The detector mass range was set to 100 to 650 amu. The temperature of the transfer line (GC to MS) was held at 250°C. Samples were injected onto the column via a split vent (split ratio 50:1) through a fused silica narrow bore injection liner packed with deactivated quartz wool; the injection port temperature was maintained at 200°C. The injection volume was 1 µl. System control, data collection and mass spectral analysis was carried out using Perkin Elmer TurboMass software (TurboMass v. 4.4).

### Glycosidase inhibition assays on Aqueous Ethanol Extract of Gallo Venere Black Rice

All enzymes and para-nitrophenyl substrates were purchased from Sigma, with the exception of beta-mannosidase which came from Megazyme. Enzymes were assayed at 27 °C in 0.1M citric acid / 0.2M disodium hydrogen phosphate buffers at the optimum pH for the enzyme. The incubation mixture consisted of 10µl enzyme solution, 10µl of 10mg/ml aqueous solution of extract and 50µl of the appropriate 5 mM para-nitrophenyl substrate made up in buffer at the optimum pH for the enzyme. The reactions were stopped by addition of 70µl 0.4M glycine (pH 10.4) during the exponential phase of the reaction, which had been determined at the beginning using uninhibited assays in which water replaced inhibitor. Final absorbances were read at 405 nm using a Versamax microplate reader (Molecular Devices). Assays were carried out in triplicate, and the values given are means of the three replicates per assay. Reference for method:- Watson, A.A., et al. (1997). Glycosidase-inhibiting pyrrolidine alkaloids from Hyacinthoides non-scripta. Phytochemistry 46 (2): 255-259

**Table 1: Mean % Glycosidase Inhibition of anion exchange resin fractionated extracts of Black rice**

| Enzyme | Source | pH | CG400 unbound | CG400 bound | Boiled IR120 bound |
|---|---|---|---|---|---|
| α-D-glucosidase | Saccharomyces cerevisiae | 6.8 | 24 | 40 | 41 |
| α-D-glucosidase | Bacillus sterothermophilus | 6.8 | <5 | 29 | 45 |
| α-D-glucosidase | rice | 4.0 | 13 | <5 | 83 |
| β-D-glucosidase | Almond (Prunus sp.) | 5 | 25 | 9 | 28 |
| α-D-galactosidase | Green coffee bean (Coffea sp.) | 6.5 | 46 | -7 | 40 |
| β-D-galactosidase | Bovine liver | 7.3 | 87 | 25 | 91 |
| α-D-mannosidase | Jack bean (Canavalia ensiformis) | 4.5 | -11 | -15 | -23 |
| β-D-mannosidase | Cellullomonas fimi | 6.5 | <5 | 19 | -5 |
| N-acetyl-β-D-glucosaminidase | Bovine kidney | 4.25 | 55 | <5 | 73 |
| N-acetyl-β-D-glucosaminidase | Jack bean | 5 | 97 | -25 | 94 |
| β-glucuronidase | Bovine liver | 5 | -9 | 7 | <5 |

### Example 2. Fagomine Content of Commercial Rice Grains

Some rice varieties are said to have a lower GI index than others, e.g. brown basmati rice.

A selection of supermarket rice grain products were extracted in 50% aq. ethanol and the amino acid fraction concentrated using cation exchange chromatography (IR120 H⁺ form). Fagomine and possibly other iminosugars were found to be present in some varieties with claims for low GI Index, e.g. brown basmati rice and Thai Jasmine and absent or considerably lower in concentration in others, e.g. long grain white rice. 1-deoxynojirmiycin (DNJ) was also present in some varieties. Glucuronidase inhibition was measured which suggests that iminosugar acids may also be present. Iminosugars may lower GI index by slowing down the degradation of complex carbohydrates in the GI tract.

**Table 2: Fagomine Content of Commercial Rice Grains**

| **Rice Type** | **Fagomine content** | **Amino acids total** |
|---|---|---|
| Brown Basmati | 1mg/100g | 53mg/100g |
| Long grain brown rice | <0.01mg/100g | 29mg/100g |
| Thai Jasmine | 0.4mg/100g | 17.5mg/100g |
| Arborio | <0.01mg/100g | 37mg/100g |
| Long grain white rice | <0.01mg/100g | 31mg/100g |
| Wild black rice | <0.01mg/100g | 321mg/100g |
| Red Camargue rice | <0.1mg/100g | 99mg/100g |

Fagomine was quantified using authentic DMDP (2,5-dihydroxymethyl-3,4-dihydroxypyrrolidine) 0.1 mg per sample) as an internal reference.

Fagomine (trimethyllsilylderivative) had a retention time of 7.5 minutes and DMDP at 8.83 minutes by GC-MS using the method described below.

### GC-MS

All samples were freeze dried before derivatisation. Trimethylsilyl (TMS) derivatives were prepared using a mixture of hexamethyldisilazane and trimethylchlorosilane in pyridine (Pierce 'Tri-Sil' silylation reagent, HMDS:TMCS:pyridine in a ratio of 2:1:10). Samples were heated at 60°C for 15 minutes and then left at room temperature for at least 60 min. Insoluble reaction products were sedimented by centrifugation, and the supernatant was transferred to fresh vials using a syringe.

Analysis was carried out by GC-MS using a Perkin Elmer Autosystem XL gas chromatograph with a high polarity fused-silica column (Varian 'Factor Four' VF-5ms column, 25 m x 0.25 mm i.d., 0.25 µm phase thickness). The carrier gas (helium) flow rate was 1 ml min-1. Trimethylsilyl- (TMS) derivatives were separated using a temperature programme that started at 160°C for 5 min, followed by a linear increase to 300°C at a rate of 10°C min-1. The temperature was held at 300°C for an additional 10 min; the total analysis time was 29 min. Electron impact mass spectrometry of the column eluant was carried out using a Perkin Elmer TurboMass Gold mass spectrometer, with a quadrupole ion filter system, which was run at 250°C constantly during analysis. The detector mass range was set to 100 to 650 amu. The temperature of the transfer line (GC to MS) was held at 250°C. Samples were injected onto the column via a split vent (split ratio 50:1) through a fused silica narrow bore injection liner packed with deactivated quartz wool; the injection port temperature was maintained at 200°C. The injection volume was 1 µl. System control, data collection and mass spectral analysis was carried out using Perkin Elmer TurboMass software (TurboMass v. 4.4).

Although the fagomine content is quite low per 100g at around 1mg in Brown Basmati Rice, the total amino acid fraction weight containing the iminosugar in that rice is not very high with an estimated 1.9% being fagomine. This makes fagomine a significant component of the low molecular weight orally available nitrogen-containing fraction. By contrast, most rice samples studied contained no fagomine (or below the detection limit of 0.03% using long grain brown rice as an example) in the amino acid fraction.

**Table 3: showing the Mean % Glycosidase Inhibition of cation exchange resin retained fractions (10mg/ml)**

| | bovine liver β-glucuronidase assay | E coli β-glucuronidase |
|---|---|---|
| Long grain white rice | 14.2 | 6.4 |
| Jasmine rice | 27.1 | 41 |

### Glycosidase inhibition assays on purchased rice grains

β-glucuronidase and the para-nitrophenyl substrate were purchased from Sigma. Enzymes were assayed at 27 °C in 0.1M citric acid / 0.2M disodium hydrogen phosphate buffers at the optimum pH for the enzyme. The incubation mixture consisted of 10µl enzyme solution, 10µl of 10mg/ml aqueous solution of extract and 50µl of the appropriate 5 mM para-nitrophenyl substrate made up in buffer at the optimum pH for the enzyme. The reactions were stopped by addition of 70µl 0.4M glycine (pH 10.4) during the exponential phase of the reaction, which had been determined at the beginning using uninhibited assays in which water replaced inhibitor. Final absorbances were read at 405 nm using a Versamax microplate reader (Molecular Devices). Assays were carried out in triplicate, and the values given are means of the three replicates per assay. The method is described in Watson, A.A., et al., 1997, Glycosidase-inhibiting pyrrolidine alkaloids from Hyacinthoides non-scripta. Phytochemistry 46 (2): 255-259,

### Example 3. Iminosuqars in a Rice Drink - Rice Dream your source of vitality

Rice Dream was purchased from Waitrose supermarket (Hain Europe N.V. Industrielaan 11 A, 9990 Maidegem, Belgium Organic 16.03.10 1 litre 29-45-075).

100ml was applied to a column (2x20cm) of cation exchange resin IR120 in the H⁺ form and the retained fraction (displaced with 2M ammonia solution) yielded 15mg of an amino acid fraction containing iminosugars.

The Rice Dream milk analysed by GC-MS as described below contained 1-deoxynojirmiycin (DNJ) at retention time (11.17 minutes) similar to glucose and with the distinctive fragmentation (tms under the conditions described) with ions at 217, 258, 348 amu. Other possible iminosugars were observed at 10.6 minutes (ions 230, 246, 290 and 302 amu) and 11.4 minutes (ions 156, 200 and 217 amu).

### GC-MS

All samples were freeze dried before derivatisation. Trimethylsilyl (TMS) derivatives were prepared using a mixture of hexamethyldisilazane and trimethylchlorosilane in pyridine (Pierce 'Tri-Sil' silylation reagent, HMDS:TMCS:pyridine in a ratio of 2:1:10). Samples were heated at 60°C for 15 minutes and then left at room temperature for at least 60 min. Insoluble reaction products were sedimented by centrifugation, and the supernatant was transferred to fresh vials using a syringe.

Analysis was carried out by GC-MS using a Perkin Elmer Autosystem XL gas chromatograph with a high polarity fused-silica column (Varian 'Factor Four' VF-5ms column, 25 m x 0.25 mm i.d., 0.25 µm phase thickness). The carrier gas (helium) flow rate was 1 ml min-1. Trimethylsilyl- (TMS) derivatives were separated using a temperature programme that started at 160°C for 5 min, followed by a linear increase to 300°C at a rate of 10°C min-1. The temperature was held at 300°C for an additional 10 min; the total analysis time was 29 min. Electron impact mass spectrometry of the column eluant was carried out using a Perkin Elmer TurboMass Gold mass spectrometer, with a quadrupole ion filter system, which was run at 250°C constantly during analysis. The detector mass range was set to 100 to 650 amu. The temperature of the transfer line (GC to MS) was held at 250°C. Samples were injected onto the column via a split vent (split ratio 50:1) through a fused silica narrow bore injection liner packed with deactivated quartz wool; the injection port temperature was maintained at 200°C. The injection volume was 1 µl. System control, data collection and mass spectral analysis was carried out using Perkin Elmer TurboMass software (TurboMass v. 4.4).

### Example 4. Iminosugars in Balance Foods Fairtrade Organic Rice Drink

The rice milk drink was bought in Waitrose Abergavenny. It was sold by Balance Foods, 1 litre, Balance Foods Ltd, Elstead GU8 6LB 06:44:24.

100ml was applied to a column (2x20cm) of cation exchange resin IR120 in the H+ form and the retained fraction (displaced with 2M ammonia solution) yielded 6.9mg of an amino acid fraction containing iminosugars.

The sample contained fagomine and a fagomine epimer with slightly longer retention time (8.3 minutes rather than 8.1 for fagomine); both compounds gave the characteristic trimethylsilyl-fragmentation pattern for fagomine under the conditions described with fragments at 144, 170 and 260 amu.

### GC-MS

All samples were freeze dried before derivatisation. Trimethylsilyl (TMS) derivatives were prepared using a mixture of hexamethyldisilazane and trimethylchlorosilane in pyridine (Pierce 'Tri-Sil' silylation reagent, HMDS:TMCS:pyridine in a ratio of 2:1:10). Samples were heated at 60°C for 15 minutes and then left at room temperature for at least 60 min. Insoluble reaction products were sedimented by centrifugation, and the supernatant was transferred to fresh vials using a syringe.

Analysis was carried out by GC-MS using a Perkin Elmer Autosystem XL gas chromatograph with a high polarity fused-silica column (Varian 'Factor Four' VF-5ms column, 25 m x 0.25 mm i.d., 0.25 µm phase thickness). The carrier gas (helium) flow rate was 1 ml min-1. Trimethylsilyl- (TMS) derivatives were separated using a temperature programme that started at 160°C for 5 min, followed by a linear increase to 300°C at a rate of 10°C min-1. The temperature was held at 300°C for an additional 10 min; the total analysis time was 29 min. Electron impact mass spectrometry of the column eluant was carried out using a Perkin Elmer TurboMass Gold mass spectrometer, with a quadrupole ion filter system, which was run at 250°C constantly during analysis. The detector mass range was set to 100 to 650 amu. The temperature of the transfer line (GC to MS) was held at 250°C. Samples were injected onto the column via a split vent (split ratio 50:1) through a fused silica narrow bore injection liner packed with deactivated quartz wool; the injection port temperature was maintained at 200°C. The injection volume was 1 µl. System control, data collection and mass spectral analysis was carried out using Perkin Elmer TurboMass software (TurboMass v. 4.4).

### Example 5. Analysis of Six Different Samples of Rice

The following samples of rice were purchased in Aberystwyth in 2012-13:

**Table 4: Rice samples**

| Code | Supplier | Brand Name | Product Name | Lot number | Expiry Date | Country |
|---|---|---|---|---|---|---|
| BBJ10 | Maeth y Meysydd Wholefood shop | Maeth y Meysydd | long grain brown rice | none | 25-Sep-13 | Italy |
| BBJ11 | Morrisons Supermarket | Morrisons | arborio rice | GS2173 | Jun-14 | Unknown |
| BBJ12 | Go East food shop | Biyori | sushi rice | none | Jan-13 | Japan |
| BBJ13 | Maeth y Meysydd Wholefood shop | Maeth y Meysydd | short grain brown rice | none | 21-Sep-13 | Italy |
| BBJ14 | Morrisons Supermarket | Tilda | wholegrain basmati rice | 13023 | Jul-14 | India |
| BBJ15 | Morrisons Supermarket | Tilda | basmati rice | 12328 | Nov-14 | India |

30g of the rice samples were finely ground and then 20g of each was soaked for at least 1 day in 50% aqueous ethanol. The suspensions were filtered and passed down IR120 columns in the H⁺ form (5x2cm).After washing with copious amounts of water; bound material was eluted with 2M ammonia solution. The solutions were taken to dryness using rotary evaporation and freeze drying, and the residues were weighed. The following weights of bound samples were obtained:-

| | |
|---|---|
| BBJ10 | 16mgs |
| BBJ11 | 5mgs |
| BBJ12 | 7mgs |
| BBJ13 | 18mgs |
| BBJ14 | 13mgs |
| BBJ15 | 7mgs |

These weights represent the amounts of amino acids and iminosugars present in 20g of each type of rice. GCMS analysis of the trimethylsilylated samples was carried out on each sample as described previously, and a peak at 7.08mins, identified as the iminosugar fagomine (by characteristic ions at 144, 170 and 260 amu) was detected in the two Basmati rice samples but in none of the others. In order to confirm this finding, much larger amounts of the other 4 rice types were subjected to GCMS but no fagomine or 1-deoxynojirimycin was detected in these samples.

### Example 6: Faqomine-related compounds in rice

D-fagomine has been reported to have potential as a dietary ingredient or functional food component to reduce the health risks associated with an excessive intake of fast-digestible carbohydrates (effective at 1 mg/kg) and can inhibit adhesion to intestinal mucosa of potentially pathogenic bacteria such as *Escherichia coli* and *Salmonella enterica* while promoting adhesion of *Lactobacillus* spp. (Gomez, L. et al., Brit. J. Nutr. 2012, 107, 1739-1746).

Further analysis was conducted to characterise compounds related to fagomine in BBJ10-15 (as listed in Example 5, above), and in a further seventh rice sample BBJ09:

| Code | Supplier | Brand Name | Product Name | Lot number | Expiry Date | Country |
|---|---|---|---|---|---|---|
| BBJ09 | Milbo Spa | Gallo Venere | Black Rice | | 26.3.15 | Italy |

The cation exchange resin-retained fractions of the 7 commercial rice samples were further fractionated using Amberlite CG400 anion exchange resin in the OH- form. Water fractions were collected followed by a 1M acetic acid wash. Basic compounds such as fagomine and DNJ are found in the water wash and acidic and neutral compounds found in the acid wash.

Samples were freeze dried and analysed as trimethylsilyderivatives by adding 30µl of Pierce TriSil to 1mg of dried sample. Analysis was carried out using by GC-MS using a Perkin Elmer Autosystem XL gas chromatograph with a high polarity fused-silica column (Varian 'Factor Four' VF-5ms column, 20 m x 0.25 mm i.d., 0.25 µm phase thickness). The carrier gas (helium) flow rate was 1 ml min-1. Trimethylsilyl- (TMS) derivatives were separated using a temperature programme that started at 160°C for 5 min, followed by a linear increase to 300°C at a rate of 10°C min-1. The temperature was held at 300°C for an additional 10 min; the total analysis time was 29 min. Electron impact mass spectrometry of the column eluant was carried out using a Perkin Elmer TurboMass Gold mass spectrometer, with a quadrupole ion filter system, which was run at 250°C constantly during analysis. The detector mass range was set to 100 to 650 amu. The temperature of the transfer line (GC to MS) was held at 250°C. Samples were injected onto the column via a split vent (split ratio 50:1) through a fused silica narrow bore injection liner packed with deactivated quartz wool; the injection port temperature was maintained at 200°C. The injection volume was 1 µl.

Furthermore, the same rice batch contained a compound tentatively identified as the N-ethyl-fagomine with retention time of 8.10 minutes and with distinctive ions (tms) at 144, 170 and 260 amu (as for fagomine) with additional ions at 198 (100%), 288, 376 (molecular ion - 15) and 391 (molecular ion).

Tilda Basmati rice and Tilda Wholegrain Basmati in addition to fagomine also contained pipecolic acid derivatives with slightly shorter retention times (4-5 minutes) tentatively identified as 3,4-dihydroxypipecolic acid, 3-hydroxypipecolic acid and 4-hydroxypipecolic acid. The compounds gave distinctive tms spectra with fragments at 172 (100%) and 130 and 156 (both 30%).

The pipecolic acids, while not being glucosidase inhibitors, are structurally related to the 3,4,5-trihydroxypipecolic acid from *Gymnema sylvestre* reported to have anti-diabetic activity (see WO2009/103953). It is probable that the pipecolic acids and fagomine are biosynthetically related. Hydroxylated pipecolic acids (for example(2R,3R,4R,5S)-3,4,5-trihydroxypiperidine-2-carboxylic acid (6-epiBR1; 2R,3R,4R,5S-Trihydroxypipecolic acid; ido-BR1 - see Bashyal et al. (1986) Tetrahedron Lett. 27: 3205-3208) have also been claimed to have anti-inflammatory activity and so rice might also have benefits related to reducing inflammatory disorders.

The pipecolic acids and N-ethyl-fagomine were not evident in the other rice grains studied.

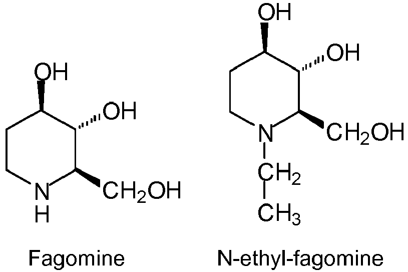

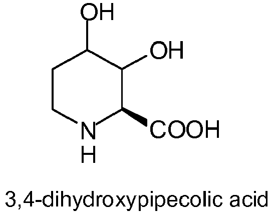

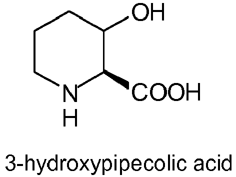

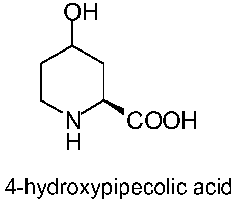

### Rat digestive enzyme assays (Table 5)

Rat intestinal acetone powder extract was purchased from Sigma (11630 lot SLBB6071V) to give results more closely related to digestion. 100 mg of the acetone powder was mixed with 1 ml cold PBS homogenised, sonicated briefly and then spun at 12K rpm for 3mins. The rat intestinal extract showed good activity with the PNP α-glucoside and PNP β-galactoside substrates. It showed even stronger activity with PNP α-galactoside substrate but much weaker activity with PNP β-glucoside. Fagomine inhibited the alpha-glucosidase activity but 3-epifagomine and 3,4-diepifagomine inhibited none of the activities measured.

The Black rice gave strongest inhibition of the digestive enzymes with not only alpha-glucosidase inhibition but also strong inhibition of alpha-galactosidase. The Black rice also gave good inhibition of the alpha-glucosidase from fractions containing no fagomine such as HS0816/78/10 which suggests that it contains other compounds inhibiting alpha-glucosidase in addition to fagomine (DNJ would also not be in this CG400 anion exchange resin retained fraction).

Both Basmati rice samples gave inhibition of the alpha-glucosidase activity. The other rice samples (BBJ10-13) were not tested in this assay.

**Table 5: glycosidase inhibitory profiles**

| | **% inhibition with substrates** | | | | | | |
|---|---|---|---|---|---|---|---|
| **Code** | **Concentration** | PNP α-D-glucoside | PNP β-D-glucoside | PNP α-D-galactoside | PNP β-D-galactoside | PNP N-acetyl-β-D-glucosaminide | PNP-glucuronide |
| BBJ09 | 10mg/ml | **66.3** | ND | 34.7 | 31 | 32.6 | ND |
| BBJ09 | 10mg/ml | 5.5 | 16.8 | **64.3** | 33 | 12.9 | 4.2 |
| BBJ09 | 5mg/ml | **69.6** | ND | 1.4 | 10.6 | 8.2 | ND |
| BBJ09 | 2mg/ml | **63.1** | 104 | 3.6 | 6.2 | 8.9 | 0.3 |
| BBJ09 | 2mg/ml | **55** | 8.9 | 0.8 | 13 | 10.5 | 1.1 |
| BBJ09 | 10mg/ml | 55.8 | 10.5 | 7 | 7 | 10.7 | 1.3 |
| BBJ14 | 10mg/ml | 27.1 | 2.9 | 0.3 | 2.1 | 19.7 | 5.5 |
| BBJ15 | 10mg/ml | 38.4 | -0.2 | 2.5 | 6.2 | 13 | 1.3 |
| fagomine | 1mg/ml | **67.1** | 4.2 | 0.4 | 2.3 | 0 | 4.5 |
| fagomine glucoside | 1mg/ml | 0.5 | -0.3 | 4.4 | 3 | 1.8 | 2.7 |
| 3,4-diepi fagomine | 1mg/ml | 38.1 | 5.8 | 3.3 | 3.6 | 10.1 | -3.8 |

It is clear from these rat digestive results that components of Black rice and Basmati rice can inhibit digestive glycosidases.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A method for selecting breeding lines and/or varieties of plants of the genus *Oryza* as a botanical source of foodstuffs of low or high GI index, the method comprising the steps of:
(a) providing seeds or vegetative parts from plants of the genus Oryza; and
(b) determining the presence or absence and/or measuring the amount of at least one polyhydroxylated alkaloid iminosugar in the seeds or vegetative parts of step (a).

2. Use of rice material from seeds of plants of the genus *Oryza* as a food or feed supplement to lower the glycaemic index (GI) value of said food or feed, wherein the rice material is selected from indica rice, japonica rice, aromatic rice, glutinous rice, brown rice, white rice, black rice, purple rice, red rice, rice bran, nonsticky rice, sticky rice, long-grained rice, short-grained rice or medium-grained rice and contains at least one endogenous polyhydroxylated alkaloid rice iminosugar, wherein said rice material is selected for a high content of said endogenous rice iminosugar.

3. The use of claim 2 wherein said food or feed is selected from: (i) a dried breakfast cereal; (ii) food for diabetics; (iii) a diet food or beverage for consumption as part of a calorie-controlled diet; (iv) bread; (v) flour; (vi) a sports beverage; (vii) a caffeine-containing beverage; (viii) a rice and/or cereal-containing snack bar; or (ix) a dried rice and/or (x) a cereal breakfast food.

4. The method of claim 1 or use of claim 2 or claim 3 wherein the at least one iminosugar is of a structural class selected from:
(a) a piperidine;
(b) a pyrroline;
(c) a pyrrolidine;
(d) a pyrrolizidine;
(e) an indolizidine;
(f) a quinolizidine;
(g) a nortropane;
(h) ring-open iminosugars;
(i) an azepane;
(j) an azetidine;
(k) an iminosugar acid (for example a (poly)hydroxypipecolic acid); and/or
(l) mixtures of any two or more of (a) to (k).

5. The method or use of claim 4 wherein the at least one iminosugar is selected from: (a) fagomine; (b) an epimer of fagomine, e.g. 3-epifagomine; (c) N-ethyl-fagomine; (d) DNJ; or (e) an iminosugar acid; or (f) combinations of (a)-(e), optionally wherein the iminosugar acid is: (a) a (poly)hydroxypipecolic acid, for example 3,4-dihydroxypipecolic acid; (b) 3-hydroxypipecolic acid; or (c) 4-hydroxypipecolic acid.

6. The method or use of any one of the preceding claims wherein the plants of the genus *Oryza* are selected from: (a) *Oryza sativa* (Asian rice); and (b) *Oryza glabaerreima* (African rice).

## Patentansprüche

1. Verfahren zum Auswählen von Zuchtlinien und/oder Sorten von Pflanzen der Gattung *Oryza* als botanische Quelle für Nahrungsmittel mit niedrigem oder hohem GI Index, wobei das Verfahren die Schritte umfasst:
(a) Bereitstellen von Samen oder vegetativen Teilen von Pflanzen der Gattung *Oryza*; und
(b) Bestimmen der Anwesenheit oder Abwesenheit und/oder Messen der Menge von mindestens einem polyhydroxylierten Alkaloid-Iminozucker in den Samen oder vegetativen Teilen von Schritt (a).

2. Verwendung von Reismaterial von Samen von Pflanzen der Gattung *Oryza* als ein Nahrungs- oder Futtermittelzusatz, um den glykämischen Index (GI) -Wert des Nahrungs- oder Futtermittels zu senken, wobei das Reismaterial ausgewählt ist aus Indica-Reis, Japonica-Reis, aromatischem Reis, Klebreis, braunem Reis, weißem Reis, schwarzem Reis, Purpurreis, rotem Reis, Reiskleie, nicht klebrigem Reis, klebrigem Reis, langkörnigem Reis, kurzkörnigem Reis oder mittelkörnigem Reis und zumindest einen endogenen polyhydroxylierten Alkaloid-Reis-Iminozucker enthält, wobei das Reismaterial für einen hohen Gehalt des endogenen Reis-Iminozuckers ausgewählt ist.

3. Verwendung nach Anspruch 2, wobei das Nahrungs- oder Futtermittel ausgewählt ist aus: (i) einer getrockneten Frühstückszerealie; (ii) Nahrung für Diabetiker; (iii) einem Diätlebensmittel oder -getränk zum Verzehr als Teil einer kalorienkontrollierten Diät; (iv) Brot; (v) Mehl; (vi) einem Sportgetränk; (vii) einem koffeinhaltigen Getränk; (viii) einem Reis und/oder Getreide enthaltenden Snackriegel; oder (ix) einem getrockneten Reis und/oder (x) einem Getreide-Frühstückslebensmittel.

4. Verfahren nach Anspruch 1 oder Verwendung nach Anspruch 2 oder Anspruch 3, wobei der mindestens eine Iminozucker aus einer Strukturklasse ausgewählt ist aus:
(a) einem Piperidin;
(b) einem Pyrrolin;
(c) einem Pyrrolidin;
(d) einem Pyrrolizidin;
(e) einem Indolizidin;
(f) einem Chinolizidin,
(g) einem Nortropan,
(h) ringoffenen Iminozuckern,
(i) einem Azepan,
(j) einem Azetidin,
(k) einer Iminozuckersäure (zum Beispiel eine (Poly)hydroxypipecolinsäure); und/oder
(l) Mischungen von zwei oder mehreren von (a) bis (k).

5. Verfahren oder Verwendung nach Anspruch 4, wobei der zumindest eine Iminozucker ausgewählt ist aus: (a) Fagomin; (b) einem Epimer von Fagomin, z.B. 3-Epifagomin; (c) N-Ethylfagomin; (d) DNJ; oder (e) einer Iminozuckersäure; oder (f) Kombinationen von (a) - (e), wobei die Iminozuckersäure optional: (a) eine (Poly)hydroxypipecolinsäure, zum Beispiel 3,4-Dihydroxypipecolinsäure; (b) 3-Hydroxypipecolinsäure; oder (c) 4-Hydroxypipecolinsäure ist.

6. Verfahren oder Verwendung nach einem der voranstehenden Ansprüche, wobei die Pflanzen der Gattung *Oryza* ausgewählt sind aus: (a) *Oryza sativa* (asiatischer Reis); und b) *Oryza glabaerreima* (afrikanischer Reis).

## Revendications

1. Procédé pour sélectionner des lignées et/ou des variétés de plantes du genre *Oryza* à titre de sources botaniques d'aliments à IG faible ou élevé, le procédé comprenant les étapes consistant à :
(a) se procurer des graines ou des parties végétatives de plantes du genre *Oryza* ; et
(b) déterminer la présence ou l'absence et/ou mesurer la quantité d'au moins un iminosucre de type alcaloïde polyhydroxylé dans les graines ou parties végétatives de l'étape (a).

2. Utilisation d'un produit de riz issu de graines de plantes du genre *Oryza* à titre d'aliment ou de complément alimentaire pour abaisser la valeur de l'index glycémique (IG) dudit aliment ou complément, dans laquelle le produit de riz est choisi parmi le riz indien, le riz japonais, le riz aromatique, le riz gluant, le riz brun, le riz blanc, le riz noir, le riz pourpre, le riz rouge, le son de riz, le riz non collant, le riz collant, le riz à grains longs, le riz à grains courts ou le riz à grains moyens et contient au moins un iminosucre de riz endogène de type alcaloïde polyhydroxylé, dans laquelle ledit produit de riz est choisi en raison de sa teneur élevée en ledit iminosucre de riz endogène.

3. Utilisation selon la revendication 2 dans laquelle ledit aliment ou complément est choisi parmi : (i) une céréale de petit-déjeuner déshydratée ; (ii) un aliment pour diabétiques ; (iii) un aliment ou une boisson diététique à consommer dans le cadre d'un régime basses calories ; (iv) le pain ; (v) la farine ; (vi) une boisson pour sportifs ; (vii) une boisson contenant de la caféine ; (viii) une collation à base de riz et/ou de céréale ; ou un aliment de petit-déjeuner à base de (ix) riz déshydraté et/ou (x) céréale.

4. Procédé selon la revendication 1 ou utilisation selon la revendication 2 ou la revendication 3 dans lequel/laquelle ledit au moins iminosucre appartient à une classe structurale choisie parmi :
(a) une pipéridine ;
(b) une pyrroline ;
(c) une pyrrolidine ;
(d) une pyrrolizidine ;
(e) une indolizidine ;
(f) une quinolizidine ;
(g) un nortropane ;
(h) les iminosucres décyclisés ;
(i) un azépane ;
(j) une azétidine ;
(k) un acide d'iminosucre (par exemple, un acide (poly)hydroxypipécolique) ; et/ou
(l) des mélanges de deux des (a) à (k) ou plus.

5. Procédé ou utilisation selon la revendication 4 dans lequel/laquelle ledit au moins iminosucre est choisi parmi : (a) la fagomine ; (b) un épimère de fagomine, p. ex. la 3-épifagomine ; (c) la N-éthyl-fagomine ; (d) la DNJ ; ou (e) un acide d'iminosucre ; ou (f) des combinaisons de (a)-(e), éventuellement dans lequel/laquelle l'acide d'iminosucre est : (a) un acide (poly)hydroxypipécolique, par exemple l'acide 3,4-dihydropipécolique ; (b) l'acide 3-hydroxypipécolique ; ou (c) l'acide 4-hydroxypipécolique.

6. Procédé ou utilisation selon l'une quelconque des revendications précédentes dans lequel/laquelle les plantes du genre *Oryza* sont choisies parmi : (a) *Oryza sativa* (riz asiatique) ; et (b) *Oryza glabaerreima* (riz africain).
